# EUROPÄISCHE PATENTSCHRIFT

(11) **EP 0 751 147 B1**
(45) Veröffentlichungstag und Bekanntmachung des Hinweises auf die Patenterteilung: **06.09.2000**
(21) Anmeldenummer: 95117643.7
(22) Anmeldetag: 04.12.1990
(51) Int. Cl.: C12N 15/30, C07K 14/45, G01N 33/577, C12Q 1/70, A61K 39/012

(54) **Toxoplasma gondii-Antigene, ihre Herstellung und ihre Verwendung**
Toxoplasma gondii antigen, its production and use
Antigène de Toxoplasma gondii, sa production et son utilisation

(30) Priorität: 08.12.1989 DE 3940598
(43) Veröffentlichungstag der Anmeldung: 02.01.1997
(62) Teilanmeldung aus: 90123152.2
(73) Patentinhaber: Dade Behring Marburg GmbH, 35001 Marburg (DE)
(72) Erfinder: Knapp, Stefan, Dr., D-35041 Marburg (DE); Ziegelmaier, Robert, Dr., D-35037 Marburg (DE); Küpper, Hans, Dr., D-35039 Marburg (DE)

(56) Entgegenhaltungen:
- EP-A- 0 301 961
- WO-A-89/05658
- WO-A-89/08700
- GENE, Bd. 85, 1989, Seiten 215-220, XP002016122 A. JOHNSON ET AL.: "Cloning extraction and nucleotide sequence of the gene fragment encoding an antigenic protion of the nucleoside triphosphate hydrolase of T. gondii"
- PROC. NATL. ACAD. SCI. USA, Bd. 86, 1989, Seiten 7537-7541, XP002016123 M. CESBRON-DELAUW ET AL.: "Molecular characterisation of a 23kd major antigen secreted by T. gondii"
- J. IMMUNOL., Bd. 141, Nr. 10, 1988, Seiten 3584-3591, XP002016124 J. BURG ET AL.: "Molecular analysis of the gene encoding the major surface antigen of T. gondii"

## Beschreibung

Die vorliegende Erfindung betrifft die Identifizierung und gentechnologische Herstellung von Toxoplasma gondii-Antigenen. Von diesem Keim wurde eine cDNA-Expressionsgenbank hergestellt. Rekombinante Klone, die von diagnostischem Interesse sind, wurden mit einem hochtitrigen anti-Toxoplasma gondii-Kaninchenserum identifiziert und isoliert.

Toxoplasma gondii (T.gondii) ist ein obligat intrazellulär parasitierender Einzeller, der den Coccidien zugeordnet wird. Der Keim besitzt ein relativ breites Wirtsspektrum und vermag neben sehr vielen Säugetieren auch den Menschen zu infizieren. Bei letzterem findet man zwei Formen, die sich physiologisch voneinander unterscheiden: "Tachyzoiten" vermehren sich vegetativ in einer Reihe verschiedener Zelltypen. Man findet diese Form ausschließlich im akuten Stadium der Infektion. Im Unterschied dazu handelt es sich bei "Bradyzoiten" um Dauerformen, die in Zellen der Herz- und Skelettmuskulatur und in Zellen des zentralen Nervensystems enzystiert persistieren und für eine dauerhafte Immunität gegenüber einer Reinfektion verantwortlich sind. Weltweit schätzt man ca. 500 Millionen Menschen, die mit T.gondii chronisch infiziert sind.

Eine T. gondii Infektion verläuft beim gesunden Erwachsenen mit Ausnahme leichter Lymphknotenschwellungen normalerweise symptomlos. In der Schwangerschaft und bei immunsupprimierten Patienten kann eine Infektion mit diesem Keim jedoch besondere Probleme bereiten. So besteht bei Schwangeren, die keinen Immunschutz gegenüber T.gondii erworben haben, die Gefahr des intrauterinen Übergangs dieser Keime. Dies führt zur Infektion des Fötus und kann Mißbildungen des Kindes oder den Abgang der Frucht zur Folge haben.

Immunsupprimierte Patienten erwerben eine akute T.gondii Infektion häufig als Folge der Reaktivierung von enzystierten "Bradyzoiten". Dies führt in den meisten Fällen zu einer zerebralen Toxoplasmose (Enzephalitis), die unter Umständen tödlich verlaufen kann. Neben der zerebralen Toxoplasmose wird T.gondii auch als Erreger von Augenkrankheiten (Chorioretinitis) genannt. Auch hier handelt es sich um Infektionen, die auf eine Reaktivierung von "Bradyzoiten" zurückgeführt werden können.

Das Krankheitsbild der Toxoplasmose bereitet dem klinisch tätigen Arzt oftmals differentialdiagnostische Schwierigkeiten, so daß bei der Diagnosefindung die Unterstützung durch Laboratoriumsuntersuchungen angestrebt wird. Der Antikörpernachweis und die Bestimmung des Titers bzw. der Titerdynamik sind darum zum wesentlichen Rüstzeug der Toxoplasmosediagnostik geworden. Verfahren zur Erfassung von Toxoplasma-spezifischen Immunglobulinen der Klasse G und M wie indirekte Immunfluoreszenez (IF), Komplementbindungsreaktion (CF), indirekte Hämagglutination (IHA), Latex-Agglutination (LA) und "Enzyme-Linked Immunoassay" (ELISA) sind im Bereich der Serodiagnostik weit verbreitet, weisen jedoch vielfach Mängel auf. So existiert unter diesen Testverfahren eine sehr starke Variation hinsichtlich Spezifität und Sensitivität. Diese Unterschiede werden in erster Linie durch die Präparation des Antigens bedingt, das für den serologischen Test eingesetzt wird. In den meisten Fällen handelt es sich um Präparationen von Gesamtzellantigen, das einen hohen Anteil an unspezifischen Zellbestandteilen enthält und für das Auftreten von falsch positiven Testergebnissen verantwortlich gemacht wird. Zusätzlich birgt die Gewinnung der Antigene aus infizierten Mäusen die Gefahr der Infektion für den Laboranten.

Im Hinblick auf Spezifität und Sensitivität eines solchen Diagnostikums wäre somit der Einsatz definierter immunreaktiver Antigene wünschenswert, die zusätzlich eine Diskriminierung zwischen IgG- und IgM-spezifischen anti-T.gondii Antikörpern zulassen sollten.

In der Literatur wurden für T.gondii eine Reihe diagnostisch interessanter Antigene beschrieben. So beschreibt Hughes in einer Zusammenfassung (Curr. Top. Microbiol. (1985), 120: 105-139) vier Hauptantigene mit Molekulargewichten von 45-, 32-, 27-und 21 Kilodalton (kD), die für die Erfassung von anti-T.gondii Antikörper der Klasse IgG potentiell geeignet sind. Handman et al. (Immunol. (1980), 40: 579-588) und Potasman et al. (J. Infect. Diseases (1986), 154: 650-657) untersuchten Verlaufsseren von akut infizierten T.gondii Patienten im Westernblot und zeigten, daß ein 35 kD Membranantigen sehr früh mit IgG Antikörper reagiert. Von Decoster et al. (Clinic. Exper. Immunol. (1988), 73: 376-382) wurden vier diagnostisch interessante Antigene mit Molekulargewichten von 105, 97, 66 und 28.5 kD beschrieben, die im Unterschied zu dem 35 kD Antigen aus dem Kulturmedium isoliert werden können und als "excreated-secreated Antigens" (ES-Antigene) bezeichnet werden. IgG-Antikörper, die mit Antigenen von 105, 97 und 28.5 kD reagieren, scheinen gute Marker für eine chronische Toxoplasmose zu sein. Ähnlich dem 35 kD Antigen werden das 97 kD Antigen und das 66 kD Antigen sehr früh von IgM-Antikörper akut infizierten Patienten erkannt. Es ist anzumerken, daß durch die Angabe eines Molekulargewichts nach elektrophoretischer Auftrennung diese Antigene nicht ausreichend charakterisiert sind, da in der Regel mehrere Proteine in einem Molekulargewichtsbereich existieren.

Ein weiterer Marker für die akute Toxoplasmose stellt ein 6 kD Antigen dar (Ehrlich et al., (1983), Infect. Immun. 41: 683-690). Im IgM-Westernblot reagiert dieses Antigen relativ stark. Bisher gibt es nur sehr wenige Daten, die über die Natur dieses Antigens Aufschluß geben könnten.

Nur sehr wenige T.gondii Antigene wurden bisher biochemisch charakterisiert. Eine Ausnahme stellt das Hauptoberflächenprotein P30 dar. Es handelt sich bei diesem Antigen um ein Glykoprotein, das über ein Glykolipid in der Membran verankert ist (Nagel et al., (1989), J. Biol. Chem. 264: 5569-5576). Die diagnostische Bedeutung dieses Antigens ist umstritten, da P30 auch mit unspezifischen Antikörpern der Klasse IgG reagiert (Potasman et al., (1986), J. Clin. Microbiol. 24: 1050-1054).

Die Isolierung und Reinigung einzelner Antigene für den Einsatz in der Serodiagnostik ist oftmals mit erheblichem Arbeitsaufwand verbunden. Sowohl die Molekulargewichtsangabe als auch die Bewertung der Immunreaktivität eines Antigens kann sich von Fall zu Fall bei konventionell gereinigtem Antigen erheblich unterscheiden. Die Klonierung und Expression solcher Antigene sowie die strukturelle Untersuchung der korrespondierenden Gene könnte nicht nur die Ausbeute an gereinigtem Antigen verbessern, sondern sollte auch zur serologischen Charakterisierung und damit zur Prüfung der diagnostischen Relevanz des Antigens beitragen. Bisher wurden die Gene dreier immunologisch interessanter T. gondii-Antigene strukturell untersucht. Die Nukleotidsequenzen dieser Antigene - es handelt sich umP30 (Burg et al., (1988), J. Immunol. 141: 3584-3591), um ein 28 kD Antigen (Prince et al., (1989), Mol. Biochem. Parasitol. 34: 3-14) und um ein 24 kD Antigen (WO 89/05658) - sind vollständig bekannt. Für letzteres wurde im Tierversuch eine Schutzwirkung vor Infektion mit T. gondii nachgewiesen.

Die vorliegende Erfindung stellt sich zur Aufgabe, definierte und zur Diagnostik und Prophylaxe geeignete Antigene von T. gondii gentechnisch herzustellen. Es ist gelungen, mit einem hochtitrigen anti-T. gondii Kaninchenserum aus einer lambda gt11-cDNA Expressionsgenbank geeignete T. gondii Genprodukte zu identifizieren. Von 8 Klonen (F2, F28, F29, F34, F45, F61, F74 und F76) wurden partielle Nukleinsäuresequenzen und daraus abgeleitete Aminosäuresequenzen bestimmt. Alle vorgenannten Klone reagieren in Westernblots mit anti-T.gondii IgG-Humanseren. Die Klone F34, F61 und F76 reagieren zusätzlich mit spezifischen Antikörpern der Klasse IgM. Die partielle Nukleotidsequenz ist in Tab. 1 angegeben, soweit ersichtlich auch die translationellen Leseraster.

F76 (Tab. 1) gehört zu einem Protein von etwa 35 kD.

Mit Hilfe der genannten partiellen Sequenzen ist es ohne weiteres möglich, die vollständigen Gene zu o.g. Teilsequenzen zu klonieren. Die in der Tabelle 1 abgebildete partielle Sequenz wurde demzufolge dazu benutzt, die kodierenden cDNA Bereiche der dazugehörigen Gene zu vervollständigen. Hierzu wurden die cDNA's F61, F34 und F76 radioaktiv markiert und als Sonden zum Screening der cDNA Genbank eingesetzt. Zur Isolierung der cDNA des Proteins P35 wurde die Sequenz aus Tab. 1, F76, eingesetzt. Rekombinante Klone, die zu diesen Sequenzen Homologien aufweisen, wurden isoliert und strukturell charakterisiert, indem die insertierten T. gondii spezifischen cDNA Bereiche sequenziert wurden. Die Nukleotidsequenz der vollständigen Strukturgenbereiche des Proteins P35 ist in der Tabelle 2 abgebildet.

Immunreaktive Teilbereiche (immunogene Teile) sind in den Beispielen 6 und 7 für P35 repräsentativ beschrieben. Andere immunogene Proteinbereiche werden in analoger Weise getestet beziehungsweise bestimmt.

Die Erfindung betrifft folglich
(a) die isolierte insertierte DNA Sequenz des vorstehend genannten Klones, einschließlich ihres Transkriptionsproduktes und restlichen Sequenzen zur Vervollständigung des Strukturgens,
(b) DNA-Strukturen und Vektoren, die diese Sequenzen ganz oder teilweise enthalten,
(c) pro- oder eukaryotische Zellen, die mit solcher DNA transformiert sind,
(d) die von solchen transformierten Zellen exprimierten Polypeptide oder immunogenen Teile davon einschließlich ihrer Verwendung zur Diagnose und Therapie oder Prophylaxe
(e) die zugehörigen Aminosäuresequenzen (AS),
(f) Antikörper gegen die Polypeptide unter (d), einschließlich ihrer Verwendung zur Diagnose und Therapie oder Prophylaxe von T. gondii-Infektionen, sowie
(g) Verfahren zur gentechnischen Herstellung der unter (d) genannten Polypeptide oder immunogenen Teilen davon.

Die Erfindung ist ferner in den Beispielen und den Ansprüchen beschrieben.

### Beispiel 1:

### Konstruktion einer lambda gt11-cDNA Expressionsgenbank von T. gondii

### 1) Isolierung von poly(A) RNA

Konfluente HEp-2-Zellkulturen wurden mit T. gondii-Keimen nach Braveny et al. (Tropenmed. Parasitologie (1978), 29: 432-434) infiziert. Ab dem 4. Tag nach Infektion wurden die Trophozoiten durch Zentrifugation des Kulturüberstandes geerntet. Die Gesamt-RNA aus ca. 500 mg pelletierter T. gondii-Zellen (Feuchtgewicht) wurde nach einer modifizierten Methode von Chomczynski and Sacchi (1987), (Analytical Biochemistry, 162: 156-159) wie folgt isoliert: Die Zellen wurden in 20 ml Lösung D (4M Guanidiniumisothiocyanat, 0.5 % Sarcosyl, 25 mM Natriumcitrat pH 7.0, 0.1 M Mercaptoethanol) lysiert und nach Zugabe von 2 ml 2 M Natriumacetat pH 4.0, 20 ml Phenol (wassergesättigt) und 4 ml Chloroform heftig geschüttelt und 20 min. auf Eis gekühlt. Nach einem Zentrifugationsschritt (30 min, 4°C, 15 000 g) wurde die RNA aus der wässrigen Phase mit einem Volumen Isopropanol eine Stunde bei 4°C gefällt und durch nachfolgende Zentrifugation (20 min, 4°C, 15 000 rpm) pelletiert. Das Pellet wurde in 600 µl Lösung D resuspendiert und die RNA anschließend durch eine 5.7 M CsCl-Lösung (3ml) zentrifugiert (12h, 35 000 rpm, 10°C). Das Pellet wurde in 500 µ l bidest. Wasser (RNAse frei) resuspendiert und die RNA erneut mit 1/10 Volumen Natriumacetat und 2 Volumen Ethanol 2h bei -20°C gefällt und durch Zentrifugation (10 min, 14 000 rpm, 4°C in der Eppendorfzentrifuge) präzipitiert. Poly(A) RNA wurde über eine oligo (dT)-Cellulose (Pharmacia) Säule (0.5 g oligo dT-Cellulose in 10 mM Tris HCI pH 7.5, 0.5M KCI) wie folgt angereichert: Die RNA wurde nach Denaturierung (70°C, 10 min) mit LiCl (Endkonzentration 0.5 M) versetzt und langsam über die oligo dT-Cellulose Säule gegeben. Nach dem Waschen der Säule mit 20 ml Bindungspuffer (10 mM Tris HCI pH 7.5, 0.5 M KCI) wurde die poly(A) RNA mit 10 ml bidest. Wasser eluiert und mit 1/20 Volumen 8 M LiCl und 2.5 Volumen Ethanol bei -20°C 4h präzipitiert und anschließend durch Zentrifugation pelletiert (6000 Upm, 4°C, 30min), in 70% Ethanol gewaschen und getrocknet.

### 2) cDNA Synthese

Die Synthese der cDNA wurde nach einer modifizierten Methode von Gubler (U. Gubler, (1988), Nucl. Acids. Res. 16: 2726) durchgeführt: Nach der Denaturierung von 5 µg poly(A) T. gondii RNA (5 min. 70°C) wird die Synthese des ersten DNA-Stranges in Gegenwart von 50 mM Tris HCI pH 8.3, 75 mM KCI, 50 mM DTT, 15 mM MgCl₂, 0.5 dNTP, 5 µg oligo dT Primer (Boehringer, Mannheim) und 800 units Reverser Transkriptase (BRL) in einem 50 µl Ansatz bei 37°C für 1 h durchgeführt. Die Reaktion wird anschließend bei 70°C 10 min. gestoppt und die Synthese des zweiten DNA-Stranges nach Zugaben von 8 µl 1 M Tris.HCl pH 7.5, 32 µl 1 M KCI, 1.6 µl 1 M MgCl₂, ,1.6 µl 1 M DTT, 50 units E.coli-DNA-Polymerasel (Boehringer Mannheim), 3.5 units RNAseH (Boehringer, Mannheim) in 320 µl Endvolumen gestartet. Der Ansatz wird 1 h bei 16°C und 1h bei 22°C inkubiert. Die cDNA wird anschließend mit zwei Volumen Ethanol und 1/10 Volumen Natriumacetat bie -70°C 10 min. gefällt, durch Zentrifugation pelletiert und getrocknet. Das Pellet wird in 100 µl T4 DNA-Polymerase Puffer (20 mM (NH₄)₂ SO₄, 50 mM Tris. HCI pH 8.8, 10 mM MgCl₂, 50 µM dNTP) resuspendiert und die Auffüllreaktion der cDNA-Enden durch Zugabe von 10 units T4 DNA-Polymerase (Boehringer, Mannheim) gestartet. Der Ansatz wird 10 min. bei 37°C inkubiert und nach Zugabe von 100 µl Phenol/ Chloroform (1:1) phenolisiert. Die cDNA Lösung wird im Anschluß durch eine Sephacryl S 200 Säule (Pharmacia) zentrifugiert. Aus dem Eluat wird die cDNA mit zwei Volumen Ethanol und 1/10 Volumen Natriumacetat gefällt, zentrifugiert und getrocknet.

### 3) Ligierung der cDNA mit EcoRI-Adapter

Die getrocknete cDNA (1 µg) wurde in 30 µl Ligierungspuffer (30 mM Tris.HCl pH 7.8, 10 mM MgCl₂, 0.5 mM ATP, 10 mM DTT) resuspendiert, mit 40 pmol EcoRI-Adapter (Promega) und 7.5 units T4 DNA Ligase versetzt und der Ansatz 15h bei 14°C inkubiert. Nach Inaktivierung der Ligase (10 min., 70°C) und anschließender Kinasebehandlung (30 min., 37°C) nach Zugabe von 4 µl Kinase-Puffer (0.7 M Tris.HCl, pH 7.6, 0.1 M MgCl₂, 50 mM DTT), 2 µl 0.1 mM ATP und 10 units T4 Polynukleotid-Kinase (Pharmacia), wird die cDNA erneut durch eine Sephacryl S 200 Säule zentrifugiert und anschließend wie oben beschrieben mit Ethanol und Natriumacetat präzipitiert.

### 4) Ligierung der cDNA mit lambda gt11-EcoRI Fragmenten, in vitro Packaging und Transfektion von lambda gt11

Für die Ligierungsreaktion wurden in einem 10 µl Ansatz (66 mM Tris.HCl pH 7.6, 6.6 mM MgCl₂, 1 mM ATP, 5 mM DTT) 1 µg dephosphorylierte lambda gt11-EcoRl-Fragmente mit ca. 50 ng kinasierter cDNA versetzt und nach Zugabe von 3 Weiss units T4 DNA Ligase (Boehringer, Mannheim) 15 h bei 14°C inkubiert. Von diesem Ansatz werden 5 µl in einer in vitro packaging Reaktion, die nach der Vorschrift des Herstellers der Packaging-Mixe (Giga Gold Mix, Stratagene) durchgeführt wurde, eingesetzt.

Der Titer an rekombinanten Phagen wurde nach Transfektion des E.coli Stammes Y1090 bestimmt. Insgesamt wurden ca. 10⁶ rekombinante Phagen erhalten.

### Beispiel 2:

### Screening der lambda gt11-Expressionsgenbank mit einem hyperimmunen Kaninchen anti-T.gondii Serum

Anti-E.coli Antikörper wurden zuächst aus dem anti-T.gondii Kaninchenserum nach bekannten Methoden adsorbiert (L.S. Osaki (1986), J. Immun. Method. 89: 213-219; Promega Biotec (1986), ProtoBlot Immunoscreening System, Technical Manual), um unspezifische Reaktionen im Immunoblot zu reduzieren. Zu diesem Zweck wurden lambda gt11-Wildtypphagen zu einer Dichte von 5 x 10³ PFU in 9 ml LB-Weichagar/0.4% Maltose/ 10mM MgSO₄ pro 90 mm Agarplatte auf insgesamt 30 LB-Agarplatten ausplattiert. Nach zwei Stunden Inkubation bei 37°C wurden die Platten mit je einem trockenen, in 10 mM IPTG (Isopropyl-β-D-thiogalactopyranosid) äquilibrierten Nitrocellulose-Rundfilter bedeckt und weitere zwei Stunden inkubiert. Anschließend wurden die Filter herumgedreht und wiederum für zwei Stunden auf dem Agar inkubiert. Die Filter wurden dann in 5% Magermilch/ TBS-Puffer (TBS: 150 mM NaCl, 50 mM Tris.HCl pH 8.0) 10 min. bei Raumtemperatur inkubiert und nach dem Transfer in 100 ml 1/100 in 5% Magermilch/TBS verdünnten Kaninchenserum vier Stunden bei Raumtemperatur inkubiert. Dieses voradsorbierte, verdünnte Serum wurde sowohl für die Screening-Experimente als auch für Westernblots verwendet. Insgesamt wurden 6 x 10⁵ rekombinante Phagen der lambda gt11-cDNA-Bank einem Screening mit diesem Serum nach der Methode von R.Y. Young und R.W. Davis (Proc. Natl. Acad. Sci. 80: 1194 (1983) unterworfen. Dazu wurden- wie oben beschrieben - Zellen einer Kultur des E.coli K12 Stammes Y1090 mit rekombinanten lambda gt11-Phagen transfiziert (3x10⁴ Phagen/100 µl Y1090 Kultur) und auf Weichagarplatten ausplattiert (insgesamt 20 Platten). Nach 2h Inkubation bei 7°C wurden die Platten mit je einem, in 10 mM IPTG getränkten, trockenen Nitrocellulose-Filter bedeckt und weiter 2h inkubiert. Nachdem die Lage der Filter auf den Agarplatten markiert wurde, wurden die Filter vorsichtig abgehoben und in 250 ml 5% Milchpulver/TBS-Puffer 10 min bei Raumtemperatur geschüttelt. Die Filter wurden dann in frischem Milchpulver/TBS-Puffer transferiert und über Nacht bei 4°C aufbewahrt.

Nach einer weiteren Inkubation der Filter in 250 ml Milchpulver/TBS-Puffer wurden diese mit 100 ml des voradsorbierten anti-T.gondii-Kaninchenserums 1h bei Raumtemperatur leicht geschüttelt. Anschließend wurden die Filter dreimal mit je 250 ml TBS 10 min. bei Raumtemperatur gewaschen und mit 250 ml 1/300 in Milchpulver/ TBS verdünnten anti-Kaninchen-IgG alkalische Phosphatase-Konjugat (Behringwerke, Marburg) eine weitere Stunde bei Raumtemperatur geschüttelt. Nach dem Waschen der Filter (dreimal mit 250 ml TBS je 10 min bei RT geschüttelt) wurden diese wiederum in 250 ml einer Substrat-Lösung für alkalische Phosphatase (200 µ g/ml 5-bromo-4-chloro-indoxyl-phosphate-p-toluidinesalt (XP), Fa. Bachem, Best.Nr.: M1205), 500 µg/ml 4 Nitrotetrazoliumchloridblau (Fa. Sigma, Best.Nr.: N6876)) 15 min. inkubiert. Seropositive Klone, erkennbar an der ringförmigen gefärbten Zone um den Phagenplaque, wurden den Regionen auf der Petrischale zugeordnet, mit einer Pasteurpipette ausgestanzt und in 1 ml SM-Puffer resuspendiert.
Positive Phagenplaques wurden über zwei weitere Screening Schritte vereinzelt. Insgesamt wurden 83 seropositive Klone isoliert. Diese Klone wurden wie folgt weiter charakterisiert.
1) Immunologische Charakterisierung der cDNA-Klone
2) Strukturelle Charakterisierung der klonierten cDNA Inserts
   a) DNA-DNA Dot blot Untersuchungen
   b) Teilsequenzierung der cDNA Inserts zur Untersuchung der offenen Leseraster
   c) Expression der klonierten cDNA's als Genfusion mit lacZ oder lacZ' (verkürztes β-Galaktosidase Derivat)

### 3) Immunologische Charakterisierung der seropositiven cDNA-Klone

Mit Hilfe "klonspezifischer" Seren (darunter sind solche Seren gemeint, die aus dem polyklonalen Kaninchenserum durch Adsorption gegen das rekombinante Fusionsprotein eines cDNA-Klons gewonnen wurden) wurden die seropositiven Klone der Genbank immunologisch charakterisiert. Diese Seren wurden nach Ozaki et al. (J. Immun. Method. 89: 213-219 (1986) wie folgt hergestellt: Jeweils 5 x 10⁴ PFU einzelner cDNA Klone wurden nach Adsorption an E.coli Y1090 Zellen in Weichagar auf LB-Platten plattiert und nach zwei Stunden Bebrütung mit je einem in 10 mM IPTG vorbehandelten Nitrozellulosefilter bedeckt und wie unter Beispiel 2 beschrieben weiterbehandelt. Pro Klon wurden jeweils drei derart vorbehandelte Filter in dem voradsorbierten Kaninchenserum vier Stunden inkubiert und dann in 50 ml TBS 10 min. gewaschen (3 Pufferwechsel). Die auf den Filtern gebundenen Antikörper wurden mit insgesamt 15 ml eines 0.1 M Glycin/HCl-Puffers (pH 2.5) 5 min. bei Raumtemperatur heruntergewaschen und mit 3 ml 1 M Tris neutralisiert. Milchpulver wurde bis zu einer Endkonzentration von 5% hinzugesetzt. Von 20 seropositiven Klone ausgetestet. Solche Klone, deren rekombinante Proteine mit einem Serum kreuzreagierten, wurden in einer Klongruppe zusammengefaßt. In Southern Dot blot Untersuchungen zeigten ³²P-markierte Insert-DNA's nur zu den Klon-DNA's Homologie, die auch aufgrund der oben beschriebenen serologischen Daten einer Gruppe zugeordnet wurden. Jeweils ein Klon einer Gruppe wurde ausgewählt und im Westernblot mit anti-T. gondii Humanseren ausgetestet. Zu diesem Zweck wurden die Insert-Fragmente der Klon-DNA's entweder in geeignete Expressionsvektoren subkloniert oder E.coli K12-Stamm Y1089 mit den betreffenden rekombinanten lambda gt11-Derivaten lysogenisiert.

### Beispiel 4:

### Expression der β-Galactosidasefusionsproteine

Zur Untersuchung der Immunreaktivität wurden die cDNA- Fragmente der lambda gt11 Klone F2, F29, F28, F34, F61, und F76 als Genfusionen mit einem verkürzten lacZ- Derivat in Vektoren der pSEM-Serie (Knapp et al., eingereicht zur Publikation) subkloniert und die Expression der Fusionsproteine in E. coli W3110 lacI^{q} L8 (Brent und Ptashne (1981) Proc. Natl. Acad. Sci., 78: 4204-4208) durch IPTG- Zugabe induziert. Zur Expression der Fusionsproteine der Klone F45 und F74 wurde der E. coli Stamm Y1089 mit beiden lambda gt11- Derivaten lysogenisiert und anschließend die Fusionsproteine nach bekannten Methoden induziert (Huynh et al. in: Glover, DNA Cloning Volume I, S. 49-78, IRL Press, Oxford (1985)). Die Proteine aus Gesamtzellextrakten wurden nach IPTG-Induktion in der SDS- PAGE (10%) elektrophoretisch aufgetrennt und auf Nitrozellulose transferiert. Die Reaktivität der rekombinanten Proteine wurde im Westernblot mit IgG- und IgM- Humanseren belegt. Die so charakterisierten Klone wurden schließlich sequenziert.

### Beispiel 5: Sequenzierung der cDNA- Fragmente

Die Sequenzierung der cDNA-Fragmente erfolgte nach der Didesoxy-Methode von Sanger (Proc.Natl. Acad.Sci. (1977), 74:5463) unter Verwendung des "KS-Primers" (Promega). Die Insert-Fragmente der Klone F2, F29, F34, F28, F45, F61, F74 und F76 wurden mit EcoRI aus rekombinanter lambda gt11-DNA herausgespalten und nach Insertion in die EcoRI-Schnittstelle des Vektors Bluescript KS in den E. coli Stamm XL1-Blue (Stratagene, San Diego) transformiert. Einzelstrang DNA dieser rekombinanten Plasmide wurde nach Infektion der Klone mit dem Helferphagen VCS nach bekannten Methoden isoliert (Stratagene, San Diego). Je nach Orientierung der klonierten Fragmente erhält man die Sequenz des 5' oder des 3' Endes der cDNA.

Die Tabelle 1 zeigt den translationellen Leseraster und partielle Nukleotidsequenzen von F76.

### Beispiel 6:

### Diagnostische Eignung des rekombinanten T. gondii Antigens rP35

Teilsequenzen aus dem Strukturgenbereich des Antigens P35 wurden unter Verwendung der pSEM-Expressionsvektoren (Knapp et al., Biotechniques (1990), 8:280) in E. coli W3110 exprimiert. Die Expressionsprodukte bestehen aus einem n-terminalen β-Galaktosidase-Derivat von 375 Aminosäuren das c-terminal einen Insert-spezifischen Fusionsteil enthält. Die Synthese der Fusionsproteine kann wie in Knapp et al. (Biotechniques (1990), 8:280) beschrieben mit IPTG induziert werden. Für die nachfolgenden Untersuchungen wurden Gesamtzellextrakte von rekombinanten E. coli W3110 Derivaten nach IPTG Induktion im SDS-PAGE aufgetrennt. Transfer der Proteine, Inkubation mit der spezifischen Serumprobe sowie Konjugatinkubation (anti Human IgG alkalische Phosphatase) und Färbung wurden nach einem Standartprotokoll durchgeführt (in: Sambrook et al.: Molecular Cloning, Cold Spring Harbor Laboratory Press (1989)). Folgende Teilbereiche der oben genannten T. Gondii Proteine wurden exprimiert:
- rP35:: Basenpaare 363 - 527*; enthalten im Hybridplasmid pPS76

(* die Koordinaten der Nukleotidsequenzen beziehen sich auf die Angaben in der Tabelle 2)

Die Reaktivität spezifischer IgG und IgM Antikörper aus Humanseren von Patienten mit akuter und chronischer T. gondii Infektion wurde in Western Blot-Experimenten untersucht. Die Ergebnisse aus diesen Untersuchungen sind in der Tabelle 3 zusammengefaßt dargestellt. So ist für den Nachweis spezifischer IgG Antikörper das Hybridprotein, rP35 geeignet. Besonders hervorzuheben ist: 25/26 Seren reagierten mit dem Hybridprotein in IgG-Western-Blots; das Fusionsprotein, rP35, reagiert ausnahmeslos mit anti T. gondii IgG-Antikörper aus akuten Seren (n=21), die einen nachweisbaren spezifischen IgM-Antikörpertiter hatten. Demnach ist rP35 als Marker für den Nachweis von anti T. gondii IgG Antikörper in der akuten Phase der Toxoplasmose besonders geeignet. Im Unterschied dazu kann das Fusionsprotein nicht für den Nachweis spezifischer IgM Antikörper eingesetzt werden: Nur 2/21 Seren von Spendern mit akuter Toxoplasmose reagierten im IgM Blot mit dem rekombinanten P35.

### Beispiel 7:

### Eignung des rekombinanten T. gondii-Proteins rP35 im ELISA

Die Reaktivität des rekombinanten T. gondii-Proteins rP35 mit spezifischen IgG-Antikörpern wurde im ELISA untersucht. Das Protein wurde als Festphasenantigen zum Beschichten von ELISA Platten eingesetzt. Das Hybridprotein wurde wie folgt aus E. coli isoliert:
Eine Übernachtkultur des rekombinanten E. coli Stammes W3110 mit den Plasmiden
pPS76 oder pPS34 wurde 1/50 in 2L L-Broth/100mg/ml Ampicillin verdünnt und bis zu einer OD600= 0.7 bei 37 C unter heftigem Schütteln angezüchtet. Nach Zugabe von IPTG (Endkonzentration 1 mM) wurden die Kulturen weitere 3h bei 37 C heftig geschüttelt, abzentrifugiert und das Zellpellet in 150mM NaCl/50mM Tris.HC1 pH8.0/ 1 mg/ml Lysozym aufgenommen und 10' bei 37 C inkubiert. Zum Aufschluß der Zellen wurde die Zellsuspension 2x in der French Press behandelt. Der Zellaufschluß wurde abzentrifugiert (10000 rpm, 10' 4 C) und das Pellet mit dem als schwerlösliche "Inclusion Bodies" vorliegenden Fusionsprotein successive mit Harnstofflösungen unterschiedlicher Konzentrationen (1M -6M Harnstoff) "gewaschen". Dabei wurde so vorgegangen, daß zunächst das Pellet in 30 ml 1 M Harnstoff/ 10mM Tris/1 mM EDTA pH8.0 (TE) bei RT 1 h gerührt wurde. Nach dem Abzentrifugieren (10000) rpm. 10', 4 C) wurde das Pellet in 2M Harnstoff wie oben beschrieben aufgenommen und inkubiert. Diese Inkubationen wurden dann mit 3M, 4M, 5M und 6M Harnstoff fortgesetzt. Die Überstände wurden nach den Zentrifugationsschritten aufbewahrt und die darin löslichen Proteine im SDS-PAGE analysiert. Für die Beschichtung der ELISA-Platten wurden solche Überstände weiterverwendet, die neben dem Fusionsprotein nur geringe Kontaminationen an E. coli Protein enthielten (ca. 75% Fusionsprotein). Diese Überstände wurden 72 h bei 4 C gegen 1M Harnstoff/0.1 % SDS dialysiert. Für die Beschichtung der ELISA Platten wurde die Proteinkonzentration der dialysierten Proben auf 2µg/ml mit PBS pH7.0 eingestellt. Die Beschichtung erfolgte bei 4 C über Nacht wobei 100µl/well eingesetzt wurden. Die Platten wurden dann 3x mit AP-Waschpuffer (Behring Best.Nr.:1353115) gewaschen, bevor die Serumproben auf den Platten aufgetragen wurden.
Adsorption von anti-E. coli Antikörper in Serumproben: Zunächst wurden anti-E. coli Antikörper aus den Serumproben entfernt. Hierzu wurden die Zellen einer E. coli W3110 Übernachtkultur abzentrifugiert und das Pellet in 5ml PBS pH7,0 resuspendiert. Die Zellen wurden durch Ultraschall (3x Beschallen Branson Sonifier, Stufe 7) lysiert und nach Zugabe von DNAse I (Endkonzentration 1µg/ml) 10' bei 37 C inkubiert.
Humanserum und Lysatantigen wurden im Verhältnis 1:1 gemischt, in PBS pH7,0 1/50 verdünnt und 30' bei RT geschüttelt. Nach dem Abzentrifugieren wurde der Überstand mit 5 % Magermild/PBS pH7.0 versetzt (Endkonzentration 1%), davon 100µl/well auf ELISA-Platten 1h bei 37 C inkubiert und 3x mit AP-Waschpuffer gewaschen. Anschließend wurden 100 µl/well des 1/70 in AP-Konjugatverdünnungspuffer (Behring Best.Nr.: 1332115) vorverdünnten anti-Human IgG AP-Konjugates (Behring Best.Nr.: OSDH 04/05) bei 37 C 1h inkubiert. Die Platten wurden 3x mit AP-Waschpuffer gewaschen und mit 100µl/well AP-Substratlösung (Behring AP-Substrattabletten Best.Nr.: OSCX 96; Behring 10 % Diethanolamin Best.Nr.: 0243115; Substratlösung: 2 Tabletten in 10ml 10% Diethanolamin) 30' bei 37 C inkubiert und die optische Dichte der Substratlösung bei 405 nm bestimmt.

In die Untersuchungen wurden 9 Seren eines Serokonverters (Patient A) mit einbezogen. Die Serumproben wurden dem Spender an folgenden Tagen entnommen: A, 9.8.1988; B, 18.8.1988; C, 29.8.1988; D, 12.10.1988; E, 2.12.1988, F, 13.1.1989; G, 28.2.1989; H, 12.5.1989; I, 17.7.1989. Die Infektion erfolgte nachweislich am 31.7.1988. Wie aus der Tab. 4 zu entnehmen ist, zeigt bereits das Humanserum B, das nach Tag 17 entnommen wurde spezifische IgG Antikörper gegen rP35. Im Unterschied dazu war diese Serumprobe in einem klassischen, nicht-rekombinanten ELISA-System (IgG-Nachweis) negativ.

Darüber hinaus wurden 30 Humanseren von Spendern mit akuter Toxoplasmose, die spezifische IgM Antikörper enthielten, im ELISA auf IgG Antikörper gegen rP35 hin untersucht. Diese Humanseren reagierten
ausnahmslos im ELISA, der das rekombinante Antigen, rP35, auf der Festphase enthielt.
Zusätzlich wurden 150 Blutspendeseren auf spezifische anti-rP35 IgG-Antikörper hin untersucht.
Die gleichen Antiseren wurden in dem Enzygnost^{R} Toxoplasmosis (IgG; Hersteller: Behringwerke AG) auf spezifische IgG-Antikörper untersucht und die Ergebnisse aus beiden Testungen miteinander verglichen. Dabei zeigte sich, daß die im Enzygnost^{R} positiven Seren auch im rP35-ELISA positiv waren. Auch bei den anti-T. gondii negativen Seren stimmten die Daten aus dem Enzygnost^{R}-ELISA und dem rP35-ELISA überein.

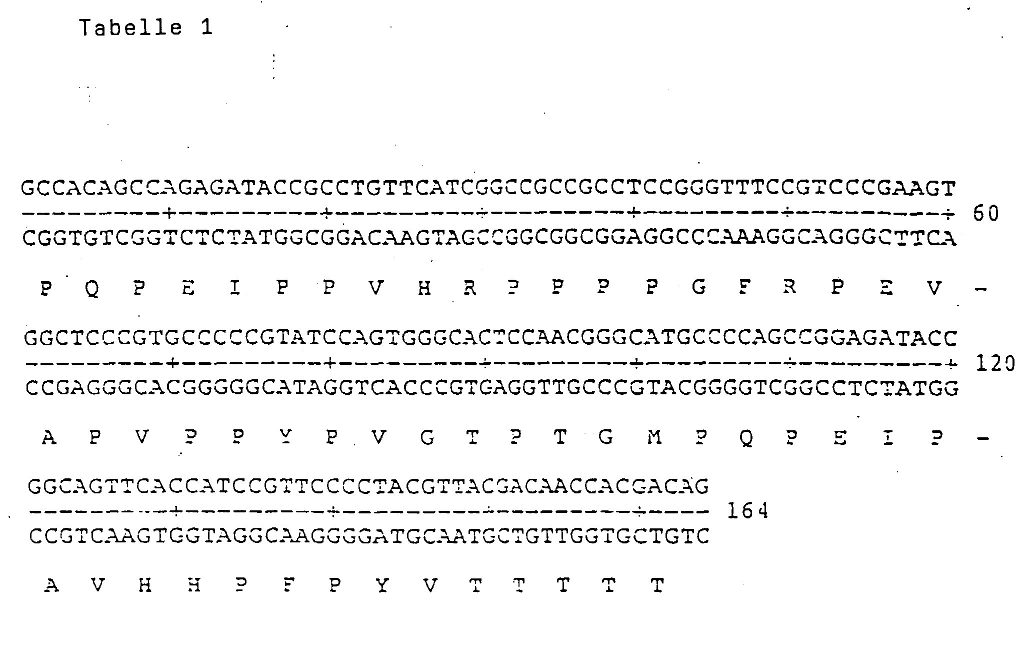

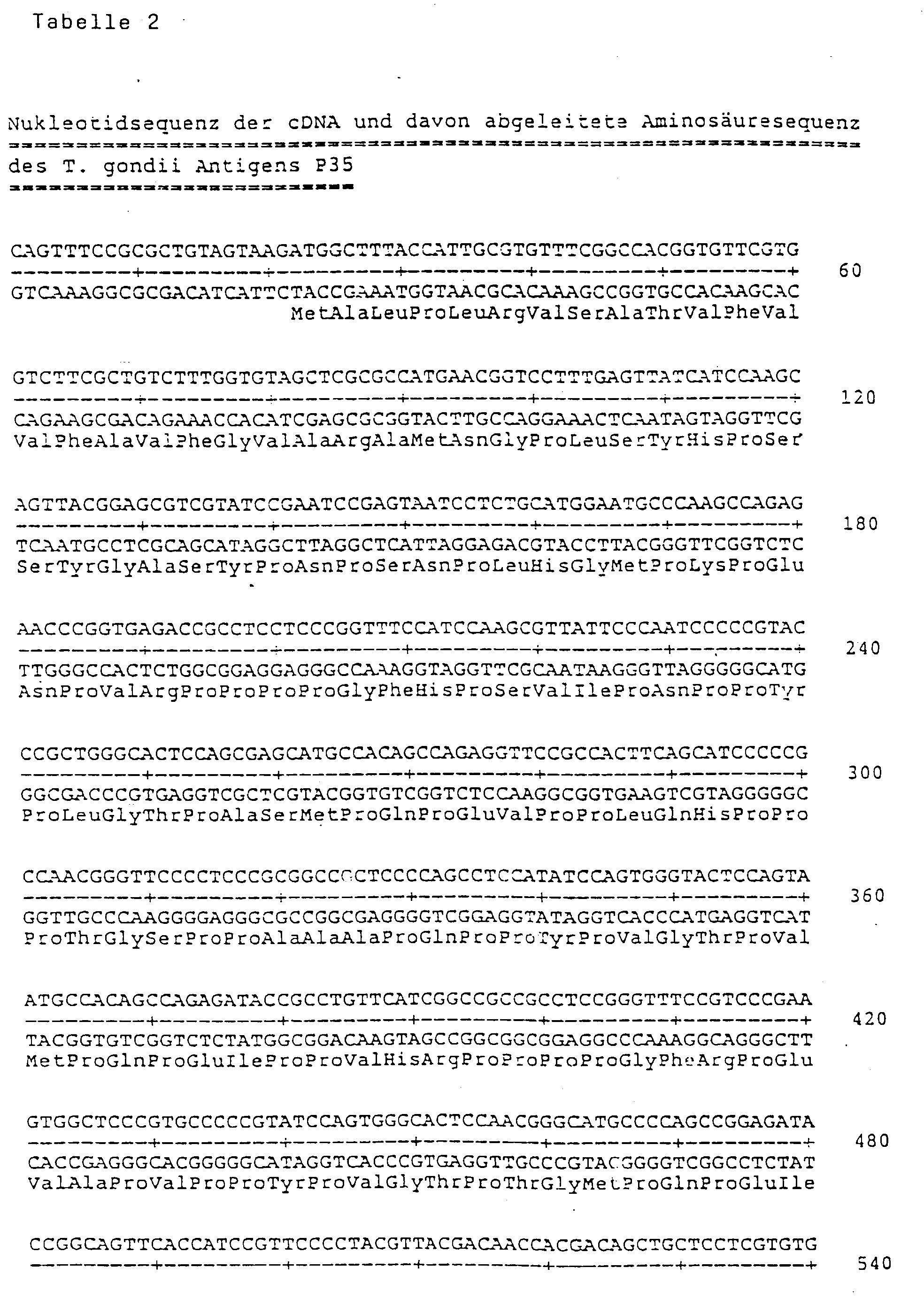

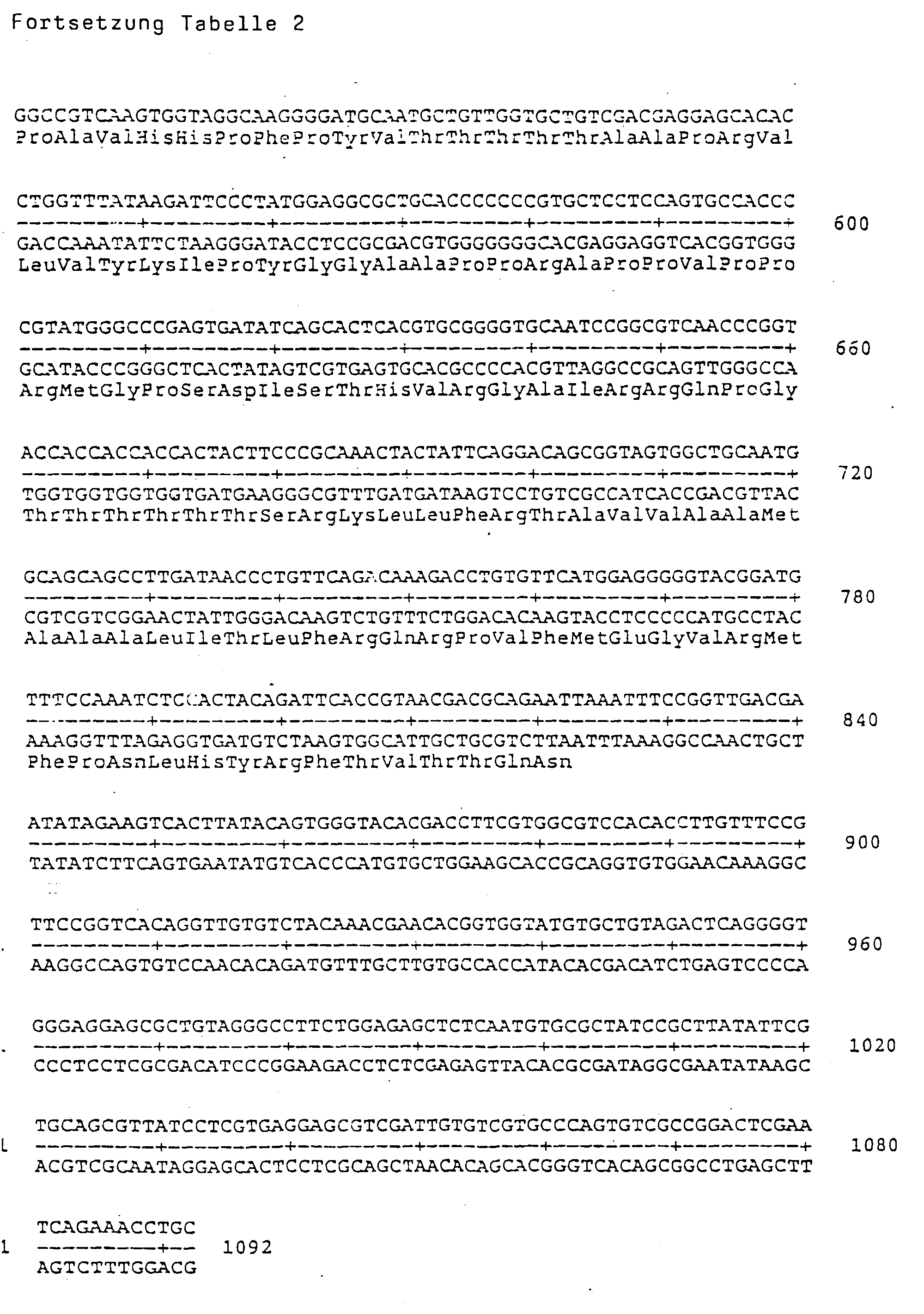

**Tabelle 3**

| **Western blot-Auswertung** | |
|---|---|
| T. gondii-Protein | r-P35 |
| Expressionsplasmid | pPS76 |
| IgG | 25/26 |
| IgM | 2/21 |

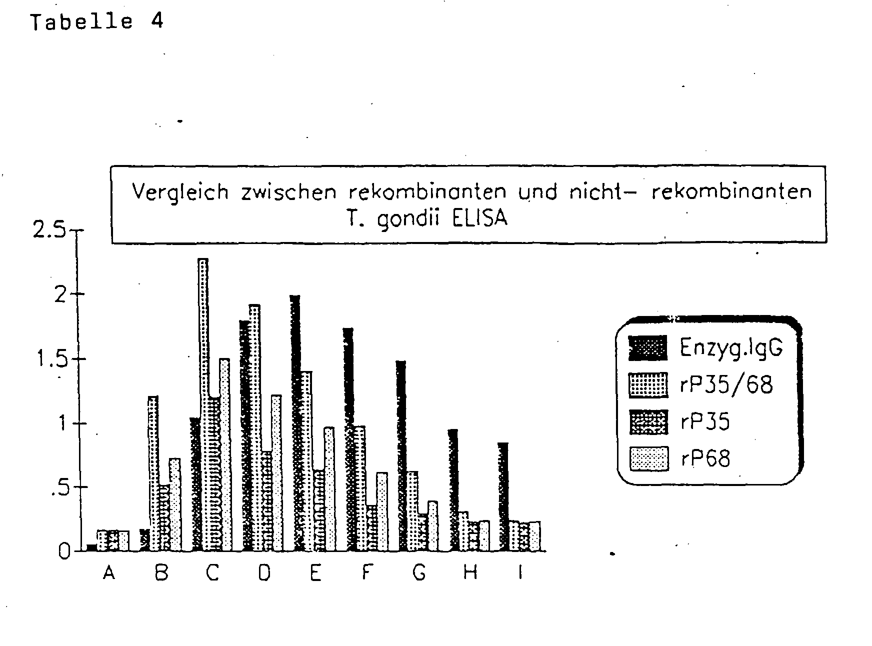

## Patentansprüche (Patentansprüche für folgende(n) Vertragsstaat(en): AT, BE, CH, LI, DE, DK, FR, GB, IT, LU, NL, SE)

1. Proteine, die Aminosäuresequenzen gemäß Tabelle 1 bzw. 2 enthalten oder von den DNA-Sequenzen besagter Tabellen kodiert werden oder immunogene Teile davon.

2. Nukleinsäuresequenzen, kodierend für Proteine nach Anspruch 1.

3. Plasmide, enthaltend Nukleinsäuresequenzen nach Anspruch 2.

4. Pro- oder eukaryotische Zellen, die mit Plasmiden nach Anspruch 3 transformiert sind.

5. Mono- oder polyklonale Antikörper gegen Proteine nach Anspruch 1.

6. Verfahren zur Herstellung von Proteinen nach Anspruch 1, dadurch gekennzeichnet, daß pro- oder eukaryotische Zellen mit Plasmiden nach Anspruch 3 transformiert werden, diese Plasmide die insertierte DNA exprimieren und die Expressionsprodukte isoliert werden.

7. Diagnostikum zum Nachweis von Toxoplasmoseinfektionen, das Proteine nach Anspruch 1 enthält.

8. Vakzine, die ein oder mehrere Proteine nach Anspruch 1 enthält.

9. Diagnostikum, enthaltend Nukleinsäuresequenzen nach Anspruch 2.

10. Diagnostikum, enthaltend Antikörper nach Anspruch 5.

11. Diagnostikum nach Anspruch 7, dadurch gekennzeichnet, daß P 35 oder jeweils immunogene Teile der genannten Proteine enthalten sind.

12. Verfahren zum Nachweis von Toxoplasmose Infektionen, dadurch gekennzeichnet, daß zu testende Körperflüssigkeiten mit einem Diagostikum nach Anspruch 7 oder Anspruch 11 gestestet werden.

13. Verwendung von Proteinen nach Anspruch 1, oder Nukleinsäuresequenzen nach Anspruch 2, oder Antikörper nach Anspruch 5 zum Nachweis von Toxoplasmoseinfektionen.

## Patentansprüche (Patentansprüche für folgende(n) Vertragsstaat(en): ES, GR)

1. Verfahren zur Herstellung von Proteinen mit Aminosäuresequenzen gemäß Tabelle 1 bzw. 2, oder von Proteinen, die von den DNA-Sequenzen besagter Tabelle kodiert werden, oder immunogenen Teilen davon, dadurch gekennzeichnet, daß pro- oder eukaryotische Zellen mit Plasmiden transformiert werden, die für die genannten Proteine kodieren, die genannten Proteine exprimiert und die Expressionsprodukte isoliert werden.

2. Verfahren zur Herstellung eines Toxoplasmosediagnostikums, dadurch gekennzeichnet, daß nach Anspruch 1 hergestellte Proteine eingesetzt werden.

3. Verfahren zur Herstellung einer Vakzine, dadurch gekennzeichnet, daß nach Anspruch 1 erzeugte Proteine eingesetzt werden.

4. Verfahren zur Herstellung mono- oder polyklonaler Antikörper, dadurch gekennzeichnet, daß nach Anspruch 1 erzeugte Proteine zur Immunisierung eingesetzt werden.

5. Verfahren zur Herstellung eines Toxoplasmosediagnostikums, dadurch gekennzeichnet, daß Nukleinsäuresequenzen nach Tabelle 1 bzw. 2 zum Einsatz kommen.

6. Verfahren nach Anspruch 2, dadurch gekennzeichnet, daß P35 oder jeweils immunogene Teile der genannten Proteine eingesetzt werden.

## Claims (Claims for the following Contracting State(s): AT, BE, CH, LI, DE, DK, FR, GB, IT, LU, NL, SE)

1. A protein which contains an amino acid sequence according to Table 1 or 2 or is encoded by a DNA sequence of said tables, or immunogenic parts thereof.

2. A nucleic acid sequence, coding for a protein as claimed in claim 1.

3. A plasmid, containing a nucleic acid sequence as claimed in claim 2.

4. Prokaryotic or eukaryotic cells which have been transformed with a plasmid as claimed in claim 3.

5. Monoclonal or polyclonal antibodies against a protein as claimed in claim 1.

6. A process for preparing a protein as claimed in claim 1, which comprises transforming prokaryotic or eukaryotic cells with a plasmid as claimed in claim 3, this plasmid expressing the inserted DNA, and the expression product being isolated.

7. A diagnostic aid for detecting toxoplasmosis infections, which contains a protein as claimed in claim 1.

8. A vaccine which contains one or more proteins as claimed in claim 1.

9. A diagnostic aid which contains nucleic acid sequences as claimed in claim 2.

10. A diagnostic aid which contains antibodies as claimed in claim 5.

11. A diagnostic aid as claimed in claim 7, which comprises P 35 or in each case immunogenic parts of said proteins.

12. A process for detecting toxoplasmosis infections, which comprises body fluids to be tested being tested with a diagnostic aid as claimed in claim 7 or claim 11.

13. The use of a protein as claimed in claim 1, or a nucleic acid sequence as claimed in claim 2, or antibody as claimed in claim 5 for detecting toxoplasmosis infections.

## Claims (Claims for the following Contracting State(s): ES, GR)

1. A process for preparing proteins having amino acid sequences according to Table 1 or 2, or proteins encoded by the DNA sequences of said table, or immunogenic parts thereof, which comprises transforming prokaryotic or eukaryotic cells with plasmids which code for the proteins mentioned, expressing the proteins mentioned and isolating the expression products.

2. A process for preparing a toxoplasmosis diagnostic aid, which comprises employing proteins prepared as claimed in claim 1.

3. A process for preparing a vaccine, which comprises employing proteins produced as claimed in claim 1.

4. A process for preparing monoclonal or polyclonal antibodies, which comprises employing proteins produced as claimed in claim 1 for immunization.

5. A process for preparing a toxoplasmosis diagnostic aid, which comprises employing nucleic acid sequences according to Table 1 or 2.

6. The process as claimed in claim 2, wherein P35 or in each case immunogenic parts of said proteins are employed.

## Revendications (Revendications pour l'(les) Etat(s) contractant(s) suivant(s): AT, BE, CH, LI, DE, DK, FR, GB, IT, LU, NL, SE)

1. Protéines qui contiennent des séquences d'aminoacides selon le tableau 1 ou 2 ou qui sont codées par les séquences d'ADN desdits tableaux, ou fragments immunogènes de celles-ci.

2. Séquences d'acide nucléique codant pour les protéines selon la revendication 1.

3. Plasmides contenant des séquences d'acide nucléique selon la revendication 2.

4. Cellules eucaryotes ou procaryotes qui sont transformées par des plasmides selon la revendication 3.

5. Anticorps monoclonaux ou polyclonaux contre les protéines selon la revendication 1.

6. Procédé de production de protéines selon la revendication 1, caractérisé en ce que l'on transforme des cellules eucaryotes ou procaryotes avec des plasmides selon la revendication 3, ces plasmides expriment l'ADN inséré et on isole les produits d'expression.

7. Agent de diagnostic pour la détection d'infections de type toxoplasmose, qui contient des protéines selon la revendication 1.

8. Vaccin qui contient une ou plusieurs protéines selon la revendication 1.

9. Agent de diagnostic contenant des séquences d'acides nucléiques selon la revendication 2.

10. Agent de diagnostic contenant des anticorps selon la revendication 5.

11. Agent de diagnostic selon la revendication 7, caractérisé en ce qu'il contient P35 ou, respectivement, des fragments immunogènes desdites protéines.

12. Procédé pour la détection d'infections de type toxoplasmose, caractérisé en ce que des liquides corporels à tester sont testés avec un agent de diagnostic selon la revendication 7 ou la revendication 11.

13. Utilisation de protéines selon la revendication 1, ou de séquences d'acide nucléique selon la revendication 2, ou d'anticorps selon la revendication 5, pour la détection d'infections de type toxoplasmose.

## Revendications (Revendications pour l'(les) Etat(s) contractant(s) suivant(s): ES, GR)

1. Procédé pour la production de protéines ayant des séquences d'aminoacides selon le tableau 1 ou 2, ou de protéines qui sont codées par les séquences d'ADN desdits tableaux, ou de fragments immunogènes de celles-ci, caractérisé en ce que des cellules procaryotes ou eucaryotes sont transformées par des plasmides qui codent pour lesdites protéines, lesdites protéines sont exprimées et on isole les produits d'expression.

2. Procédé pour la préparation d'un agent de diagnostic de la toxoplasmose, caractérisé en ce que l'on utilise des protéines produites selon la revendication 1.

3. Procédé pour la fabrication d'un vaccin, caractérisé en ce que l'on utilise des protéines produites selon la revendication 1.

4. Procédé la production d'anticorps monoclonaux ou polyclonaux, caractérisé en ce que l'on utilise pour l'immunisation des protéines produites selon la revendication 1.

5. Procédé pour la préparation d'un agent de diagnostic de la toxoplasmose, caractérisé en ce que l'on utilise des séquences d'acide nucléique selon le tableau 1 ou 2.

6. Procédé selon la revendication 2, caractérisé en ce que l'on utilise P35 ou, respectivement, des fragments immunogènes de ladite protéine.


### SEQUENCE LISTING

(1) GENERAL INFORMATION:
   (i) APPLICANT:
      (A) NAME: Behringwerke Aktiengesellschaft
      (B) STREET: Emil-von-Behring-Str. 76
      (C) CITY: Marburg
      (E) COUNTRY: Germany
      (F) POSTAL CODE (ZIP): 35041
      (G) TELEPHONE: 06421-39-2205
      (H) TELEFAX: 06421-39-4558
   (ii) TITLE OF INVENTION: Toxoplasma gondii-Antigene, ihre Herstellung und ihre Verwendung
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (vi) PRIOR APPLICATION DATA:
      (A) APPLICATION NUMBER: EP 90123152.2-2399
      (B) FILING DATE: 04-DEC-1990
(2) INFORMATION FOR SEQ ID NO: 1:
   (i) SEQUENCE CHARACTERISTICS:
      (A) LENGTH: 164 base pairs
      (B) TYPE: nucleic acid
      (C) STRANDEDNESS: double
      (D) TOPOLOGY: linear
   (ii) MOLECULE TYPE: cDNA
   (ix) FEATURE:
      (A) NAME/KEY: CDS
      (B) LOCATION:2..163
   (xi) SEQUENCE DESCRIPTION: SEQ ID NO: 1:
   G CCA CAG CCA GAG ATA CCG CCT GTT CAT CGG CCG CCG CCT CCG GGT          46
     Pro Gln Pro Glu Ile Pro Pro Val His Arg Pro Pro Pro Pro Gly
       1               5                  10                  15
   TTC CGT CCC GAA GTG GCT CCC GTG CCC CCG TAT CCA GTG GGC ACT CCA        94
   Phe Arg Pro Glu Val Ala Pro Val Pro Pro Tyr Pro Val Gly Thr Pro
                    20                  25                  30
   ACG GGC ATG CCC CAG CCG GAG ATA CCG GCA GTT CAC CAT CCG TTC CCC       142
   Thr Gly Met Pro Gln Pro Glu Ile Pro Ala Val His His Pro Phe Pro
                35                  40                  45
   TAC GTT ACG ACA ACC ACG ACA G                                         164
   Tyr Val Thr Thr Thr Thr Thr
            50
(2) INFORMATION FOR SEQ ID NO: 2:
   (i) SEQUENCE CHARACTERISTICS:
      (A) LENGTH: 54 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Pro Gln Pro Glu Ile Pro Pro Val His Arg Pro Pro Pro Pro Gly Phe
    1               5                  10                  15
   Arg Pro Glu Val Ala Pro Val Pro Pro Tyr Pro Val Gly Thr Pro Thr
               20                  25                  30
   Gly Met Pro Gln Pro Glu Ile Pro Ala Val His His Pro Phe Pro Tyr
           35                  40                  45
   Val Thr Thr Thr Thr Thr
       50
(2) INFORMATION FOR SEQ ID NO: 3:
   (i) SEQUENCE CHARACTERISTICS:
      (A) LENGTH: 1092 base pairs
      (B) TYPE: nucleic acid
      (C) STRANDEDNESS: double
      (D) TOPOLOGY: linear
   (ii) MOLECULE TYPE: cDNA
   (ix) FEATURE:
      (A) NAME/KEY: CDS
      (B) LOCATION:22..823
   (xi) SEQUENCE DESCRIPTION: SEQ ID NO: 3:
   CAGTTTCCGC GCTGTAGTAA G ATG GCT TTA CCA TTG CGT GTT TCG GCC ACG        51
                           Met Ala Leu Pro Leu Arg Val Ser Ala Thr
                            55                  60
   GTG TTC GTG GTC TTC GCT GTC TTT GGT GTA GCT CGC GCC ATG AAC GGT        99
   Val Phe Val Val Phe Ala Val Phe Gly Val Ala Arg Ala Met Asn Gly
    65                  70                  75                  80
   CCT TTG AGT TAT CAT CCA AGC AGT TAC GGA GCG TCG TAT CCG AAT CCG       147
   Pro Leu Ser Tyr His Pro Ser Ser Tyr Gly Ala Ser Tyr Pro Asn Pro
                    85                  90                  95
   AGT AAT CCT CTG CAT GGA ATG CCC AAG CCA GAG AAC CCG GTG AGA CCG       195
   Ser Asn Pro Leu His Gly Met Pro Lys Pro Glu Asn Pro Val Arg Pro
               100                 105                 110
   CCT CCT CCC GGT TTC CAT CCA AGC GTT ATT CCC AAT CCC CCG TAC CCG       243
   Pro Pro Pro Gly Phe His Pro Ser Val Ile Pro Asn Pro Pro Tyr Pro
           115                 120                 125
   CTG GGC ACT CCA GCG AGC ATG CCA CAG CCA GAG GTT CCG CCA CTT CAG       291
   Leu Gly Thr Pro Ala Ser Met Pro Gln Pro Glu Val Pro Pro Leu Gln
       130                 135                 140
   CAT CCC CCG CCA ACG GGT TCC CCT CCC GCG GCC GCT CCC CAG CCT CCA       339
   His Pro Pro Pro Thr Gly Ser Pro Pro Ala Ala Ala Pro Gln Pro Pro
   145                 150                 155                 160
   TAT CCA GTG GGT ACT CCA GTA ATG CCA CAG CCA GAG ATA CCG CCT GTT       387
   Tyr Pro Val Gly Thr Pro Val Met Pro Gln Pro Glu Ile Pro Pro Val
                   165                 170                 175
   CAT CGG CCG CCG CCT CCG GGT TTC CGT CCC GAA GTG GCT CCC GTG CCC        435
   His Arg Pro Pro Pro Pro Gly Phe Arg Pro Glu Val Ala Pro Val Pro
               180                 185                 190
   CCG TAT CCA GTG GGC ACT CCA ACG GGC ATG CCC CAG CCG GAG ATA CCG        483
   Pro Tyr Pro Val Gly Thr Pro Thr Gly Met Pro Gln Pro Glu Ile Pro
           195                 200                 205
   GCA GTT CAC CAT CCG TTC CCC TAC GTT ACG ACA ACC ACG ACA GCT GCT        531
   Ala Val His His Pro Phe Pro Tyr Val Thr Thr Thr Thr Thr Ala Ala
       210                 215                 220
   CCT CGT GTG CTG GTT TAT AAG ATT CCC TAT GGA GGC GCT GCA CCC CCC        579
   Pro Arg Val Leu Val Tyr Lys Ile Pro Tyr Gly Gly Ala Ala Pro Pro
   225                 230                 235                 240
   CGT GCT CCT CCA GTG CCA CCC CGT ATG GGC CCG AGT GAT ATC AGC ACT        627
   Arg Ala Pro Pro Val Pro Pro Arg Met Gly Pro Ser Asp Ile Ser Thr
                   245                 250                 255
   CAC GTG CGG GGT GCA ATC CGG CGT CAA CCC GGT ACC ACC ACC ACC ACT        675
   His Val Arg Gly Ala Ile Arg Arg Gln Pro Gly Thr Thr Thr Thr Thr
               260                 265                 270
   ACT TCC CGC AAA CTA CTA TTC AGG ACA GCG GTA GTG GCT GCA ATG GCA        723
   Thr Ser Arg Lys Leu Leu Phe Arg Thr Ala Val Val Ala Ala Met Ala
           275                 280                 285
   GCA GCC TTG ATA ACC CTG TTC AGA CAA AGA CCT GTG TTC ATG GAG GGG        771
   Ala Ala Leu Ile Thr Leu Phe Arg Gln Arg Pro Val Phe Met Glu Gly
       290                 295                 300
   GTA CGG ATG TTT CCA AAT CTC CAC TAC AGA TTC ACC GTA ACG ACG CAG        819
   Val Arg Met Phe Pro Asn Leu His Tyr Arg Phe Thr Val Thr Thr Gln
   305                 310                 315                 320
   AAT T AAATTTCCGG TTGACGAATA TAGAAGTCAC TTATACAGTG GGTACACGAC           873
   Asn
   CTTCGTGGCG TCCACACCTT GTTTCCGTTC CGGTCACAGG TTGTGTCTAC AAACGAACAC      933
   GGTGGTATGT GCTGTAGACT CAGGGGTGGG AGGAGCGCTG TAGGGCCTTC TGGAGAGCTC      993
   TCAATGTGCG CTATCCGCTT ATATTCGTGC AGCGTTATCC TCGTGAGGAG CGTCGATTGT     1053
   GTCGTGCCCA GTGTCGCCGG ACTCGAATCA GAAACCTGC                            1092
(2) INFORMATION FOR SEQ ID NO: 4:
   (i) SEQUENCE CHARACTERISTICS:
      (A) LENGTH: 267 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Met Ala Leu Pro Leu Arg Val Ser Ala Thr Val Phe Val Val Phe Ala
    1               5                  10                  15
   Val Phe Gly Val Ala Arg Ala Met Asn Gly Pro Leu Ser Tyr His Pro
               20                  25                  30
   Ser Ser Tyr Gly Ala Ser Tyr Pro Asn Pro Ser Asn Pro Leu His Gly
           35                  40                  45
   Met Pro Lys Pro Glu Asn Pro Val Arg Pro Pro Pro Pro Gly Phe His
       50                  55                  60
   Pro Ser Val Ile Pro Asn Pro Pro Tyr Pro Leu Gly Thr Pro Ala Ser
   65                  70                  75                  80
   Met Pro Gln Pro Glu Val Pro Pro Leu Gln His Pro Pro Pro Thr Gly
                   85                  90                  95
   Ser Pro Pro Ala Ala Ala Pro Gln Pro Pro Tyr Pro Val Gly Thr Pro
               100                 105                 110
   Val Met Pro Gln Pro Glu Ile Pro Pro Val His Arg Pro Pro Pro Pro
           115                 120                 125
   Gly Phe Arg Pro Glu Val Ala Pro Val Pro Pro Tyr Pro Val Gly Thr
       130                 135                 140
   Pro Thr Gly Met Pro Gln Pro Glu Ile Pro Ala Val His His Pro Phe
   145                 150                 155                 160
   Pro Tyr Val Thr Thr Thr Thr Thr Ala Ala Pro Arg Val Leu Val Tyr
                   165                 170                 175
   Lys Ile Pro Tyr Gly Gly Ala Ala Pro Pro Arg Ala Pro Pro Val Pro
               180                 185                 190
   Pro Arg Met Gly Pro Ser Asp Ile Ser Thr His Val Arg Gly Ala Ile
           195                 200                 205
   Arg Arg Gln Pro Gly Thr Thr Thr Thr Thr Thr Ser Arg Lys Leu Leu
       210                 215                 220
   Phe Arg Thr Ala Val Val Ala Ala Met Ala Ala Ala Leu Ile Thr Leu
   225                 230                 235                 240
   Phe Arg Gln Arg Pro Val Phe Met Glu Gly Val Arg Met Phe Pro Asn
                   245                 250                 255
   Leu His Tyr Arg Phe Thr Val Thr Thr Gln Asn
               260                 265